# EUROPEAN PATENT APPLICATION

(11) **EP 3 156 801 A1**
(43) Date of publication of application: **19.04.2017**
(21) Application number: 15189526.5
(22) Date of filing: 13.10.2015
(51) Int. Cl.: G01N 33/68, A61K 38/17

(54) **SICAM-1 AS BIOMARKER FOR PAIN AND PAIN INTENSITY**

(71) Applicant: Ludwig-Maximilians-Universität München, 80539 München (DE)
(72) Inventor: Luchting, Benjamin Sebastian, 82343 Pöcking (DE); Hinske, Ludwig Christian Giuseppe, 82061 Neuried (DE); Azad, Shahnaz Christina, 80336 München (DE)
(74) Representative: Engelhard, Markus

(57) **Abstract**

The present invention relates to soluble intercellular adhesion molecule-1 (sICAM-1) for use in the diagnosis, monitoring and/or therapy control of pain. The present invention relates to sICAM-1 as biomarker for pain intensity and its use in pain management as well as in point-of-care methods.

SEQUENCE LISTING

<110>  Ludwig-Maximilians-Universität München

<120>  sICAM-1 as biomarker for pain and pain intensity

<130>  U30655EP

<160>  2

<170>  PatentIn version 3.5

<210>  1
<211>  1443
<212>  DNA
<213>  Homo sapiens

<400>  1
atggctccca gcagcccccg gcccgcgctg cccgcactcc tggtcctgct cggggctctg      60

ttcccaggac ctggcaatgc ccagacatct gtgtccccct caaaagtcat cctgccccgg     120

ggaggctccg tgctggtgac atgcagcacc tcctgtgacc agcccaagtt gttgggcata     180

gagaccccgt tgcctaaaaa ggagttgctc ctgcctggga acaaccggaa ggtgtatgaa     240

ctgagcaatg tgcaagaaga tagccaacca atgtgctatt caaactgccc tgatgggcag     300

tcaacagcta aaaccttcct caccgtgtac tggactccag aacgggtgga actggcaccc     360

ctcccctctt ggcagccagt gggcaagaac cttaccctac gctgccaggt ggagggtggg     420

gcaccccggg ccaacctcac cgtggtgctg ctccgtgggg agaaggagct gaaacgggag     480

ccagctgtgg gggagcccgc tgaggtcacg accacggtgc tggtgaggag agatcaccat     540

ggagccaatt tctcgtgccg cactgaactg gacctgcggc cccaagggct ggagctgttt     600

gagaacacct cggcccccta ccagctccag acctttgtcc tgccagcgac tcccccacaa     660

cttgtcagcc cccgggtcct agaggtggac acgcagggga ccgtggtctg ttccctggac     720

gggctgttcc cagtctcgga ggcccaggtc cacctggcac tgggggacca gaggttgaac     780

cccacagtca cctatggcaa cgactccttc tcggccaagg cctcagtcag tgtgaccgca     840

gaggacgagg gcacccagcg gctgacgtgt gcagtaatac tggggaacca gagccaggag     900

acactgcaga cagtgaccat ctacagcttt ccggcgccca acgtgattct gacgaagcca     960

gaggtctcag aagggaccga ggtgacagtg aagtgtgagg cccaccctag agccaaggtg    1020

acgctgaatg gggttccagc ccagccactg ggcccgaggg cccagctcct gctgaaggcc    1080

accccagagg acaacgggcg cagcttctcc tgctctgcaa ccctggaggt ggccggccag    1140

cttatacaca agaaccagac ccgggagctt cgtgtcctgt atggcccccg actggacgag    1200

agggattgtc cgggaaactg gacgtggcca gaaaattccc agcagactcc aatgtgccag    1260

gcttggggga acccattgcc cgagctcaag tgtctaaagg atggcacttt cccactgccc    1320

atcggggaat cagtgactgt cactcgagat cttgagggca cctacctctg tcgggccagg    1380

agcactcaag gggaggtcac ccgcaagccc cccggtatga gattgtcatc atcactgtgg     1440

tag                                                                   1443

<210>  2
<211>  480
<212>  PRT
<213>  Homo sapiens

<400>  2

Met Ala Pro Ser Ser Pro Arg Pro Ala Leu Pro Ala Leu Leu Val Leu
1               5                   10                  15

Leu Gly Ala Leu Phe Pro Gly Pro Gly Asn Ala Gln Thr Ser Val Ser
            20                  25                  30

Pro Ser Lys Val Ile Leu Pro Arg Gly Gly Ser Val Leu Val Thr Cys
        35                  40                  45

Ser Thr Ser Cys Asp Gln Pro Lys Leu Leu Gly Ile Glu Thr Pro Leu
    50                  55                  60

Pro Lys Lys Glu Leu Leu Leu Pro Gly Asn Asn Arg Lys Val Tyr Glu
65                  70                  75                  80

Leu Ser Asn Val Gln Glu Asp Ser Gln Pro Met Cys Tyr Ser Asn Cys
                85                  90                  95

Pro Asp Gly Gln Ser Thr Ala Lys Thr Phe Leu Thr Val Tyr Trp Thr
            100                 105                 110

Pro Glu Arg Val Glu Leu Ala Pro Leu Pro Ser Trp Gln Pro Val Gly
        115                 120                 125

Lys Asn Leu Thr Leu Arg Cys Gln Val Glu Gly Gly Ala Pro Arg Ala
    130                 135                 140

Asn Leu Thr Val Val Leu Leu Arg Gly Glu Lys Glu Leu Lys Arg Glu
145                 150                 155                 160

Pro Ala Val Gly Glu Pro Ala Glu Val Thr Thr Thr Val Leu Val Arg
                165                 170                 175

Arg Asp His His Gly Ala Asn Phe Ser Cys Arg Thr Glu Leu Asp Leu
            180                 185                 190

Arg Pro Gln Gly Leu Glu Leu Phe Glu Asn Thr Ser Ala Pro Tyr Gln
        195                 200                 205

Leu Gln Thr Phe Val Leu Pro Ala Thr Pro Pro Gln Leu Val Ser Pro
    210                 215                 220

Arg Val Leu Glu Val Asp Thr Gln Gly Thr Val Val Cys Ser Leu Asp
225                 230                 235                 240

Gly Leu Phe Pro Val Ser Glu Ala Gln Val His Leu Ala Leu Gly Asp
                245                 250                 255

Gln Arg Leu Asn Pro Thr Val Thr Tyr Gly Asn Asp Ser Phe Ser Ala
            260                 265                 270

Lys Ala Ser Val Ser Val Thr Ala Glu Asp Glu Gly Thr Gln Arg Leu
        275                 280                 285

Thr Cys Ala Val Ile Leu Gly Asn Gln Ser Gln Glu Thr Leu Gln Thr
    290                 295                 300

Val Thr Ile Tyr Ser Phe Pro Ala Pro Asn Val Ile Leu Thr Lys Pro
305                 310                 315                 320

Glu Val Ser Glu Gly Thr Glu Val Thr Val Lys Cys Glu Ala His Pro
                325                 330                 335

Arg Ala Lys Val Thr Leu Asn Gly Val Pro Ala Gln Pro Leu Gly Pro
            340                 345                 350

Arg Ala Gln Leu Leu Leu Lys Ala Thr Pro Glu Asp Asn Gly Arg Ser
        355                 360                 365

Phe Ser Cys Ser Ala Thr Leu Glu Val Ala Gly Gln Leu Ile His Lys
    370                 375                 380

Asn Gln Thr Arg Glu Leu Arg Val Leu Tyr Gly Pro Arg Leu Asp Glu
385                 390                 395                 400

Arg Asp Cys Pro Gly Asn Trp Thr Trp Pro Glu Asn Ser Gln Gln Thr
                405                 410                 415

Pro Met Cys Gln Ala Trp Gly Asn Pro Leu Pro Glu Leu Lys Cys Leu
            420                 425                 430

Lys Asp Gly Thr Phe Pro Leu Pro Ile Gly Glu Ser Val Thr Val Thr
        435                 440                 445

Arg Asp Leu Glu Gly Thr Tyr Leu Cys Arg Ala Arg Ser Thr Gln Gly

450                 455                 460

Glu Val Thr Arg Lys Pro Pro Gly Met Arg Leu Ser Ser Ser Leu Trp
465                 470                 475                 480

## Description

The present invention relates to soluble intercellular adhesion molecule-1 (sICAM-1) for use in the diagnosis, monitoring and/or therapy control of pain. The present invention relates to sICAM-1 as biomarker for pain intensity and its use in pain management as well as in point-of-care methods.

### BACKGROUND OF THE INVENTION

Pain is an unpleasant sensation meant to alert the body in reaction to physical stimulus or damage. For thousands of years, people have produced and used analgesic agents, such as herbal extracts, opium, and alcohol, in order to make pain more tolerable. Despite the long history of successful treatment of pain, diagnostic measures still mainly rely on patients' self-reporting and unspecific clinical features such as overall appearance, heart-rate, and blood pressure. Consequently, pain therapy becomes a guessing game, when a patient is unable to reliably self-report her or his pain. It has been estimated that the prevalence of undertrcated pain may be as high as 80% in older adults in long term care facilities and patients with severe dementia (Barkin *et al.,* 2005; Zwakhalen *et al.,* 2006). Moreover, several studies have demonstrated that pain is experienced by the overwhelming majority of intubated patients in intensive care units (Joffe *et al.,* 2013). In these patients, poor pain management is accompanied by prolonged length of stay, duration of mechanical ventilation, increased rates of nosocomial infections, and the development of chronic pain and post-traumatic stress disorder (Payen *et al.,* 2009; Chanques *et al.,* 2006). Therefore, the lack of non-verbal avenues to measure pain impacts patients' well-being and healthcare costs greatly.

Also for routinely performed anesthesia, the anesthesiologist has no value he could measure which would help to assess the pain intensity the patient experiences while under the anesthetic. The three keystones of each narcosis are *(1)* hypnosis ("unconsciousness"), *(2)* analgesia ("no pain") and *(3)* relaxation ("reduction of muscle tonus"). For controlling hypnosis *(1),* there are measurement methods which quantify in-depth of anaesthesia by continually determining and analyzing the brain waves. Thereby, potential wake phenomena during anesthesia as well as potential overdosing of hypnotically functioning drugs are to be prevented (Ecallier *et al.,* 2014). Also muscle relaxation *(3)* can routinely be monitored and controlled via neuromuscular monitoring (see Lien and Kopman, 2014).

However, for analyzing the pain intensity there exists not one method enabling the anesthesiologist to register potential pain. Thus, when performing anesthesia, an overdosing or underdosing of pain medication occurs rather often. The length of narcosis is prolonged at most (i.e. delayed wake-up due to overdosing), or, at worst, an increased danger of experienced pain events (awareness), development of chronic pain (pain memory) or strong pain after narcosis (due to underdosing).

Recent studies have linked inflammatory molecules to pain pathophysiology (Ellis *et al.,* 2013; Wang *et al.,* 2008; Lees *et al.,* 2013; Alexander *et al.,* 2012; Sun *et al.,* 2013; Koch *et al.,* 2007). However, none of these studies was designed to identify a surrogate marker for pain intensity that could help in guiding therapy.

In summary, there is a plethora of clinical situations where an easy and objective assessment of a patient's pain status would be of immense help. To date, no such method exists.

There is a need in the art for improved means and methods for assessing pain and/or pain intensity, which further allow simple assessment.

### SUMMARY OF THE INVENTION

According to the present invention this object is solved by soluble intercellular adhesion molecule-1 (sICAM-1) for use in the diagnosis of pain.

According to the present invention this object is solved by a method for the diagnosis, monitoring and/or therapy control of pain, comprising the step of
determining the concentration or level of sICAM-1 in patient specimen.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of " 105 to 120 ng/ml" should be interpreted to include not only the explicitly recited values of 105 to 120, but also include individual values and sub-ranges within the indicated range. Thus, included in this numerical range are individual values such as 105, 106, 107... 118, 119, 120 and sub-ranges such as from 110 to 115, from 110 to 120, from 105 to 115, from 105 to 110, etc. This same principle applies to ranges reciting only one numerical value, such as "about 112.8 ng/ml". Furthermore, such an interpretation should apply regardless of the breadth of the range or the characteristics being described. Also it is to be understood that ranges may differ depending on the institute/facility where the measurements are being performed, methodology of measurement, type of tissue, and technique of tissue collection.

### sICAM as pain marker

The present invention provides soluble intercellular adhesion molecule-1 (sICAM-1) as (bio)marker for pain and pain -intensity

ICAM-1 (Intercellular Adhesion Molecule 1) also known as CD54 (Cluster of Differentiation 54) is a protein that in humans is encoded by the *ICAM1* gene (located on chromosome 19, Genbank Accession No.: NG_012083).

ICAM-1 is a member of the immunoglobulin superfamily. ICAM-1 is a transmembrane protein possessing an N-terminal extracellular domain, a single transmembrane domain, and a C-terminal cytoplasmic domain. The structure of ICAM-1 is characterized by heavy glycosylation, and the protein's extracellular domain is composed of multiple loops created by disulfide bridges within the protein. The dominant secondary structure of the protein is the beta sheet, suggesting the presence of dimerization domains within ICAM-1.

### Soluble ICAM-1 (sICAM-1):

ICAM-1 and sICAM-1 have substantial similarity in that they share the first 442 N-terminal amino acids of the extracellular domain. sICAM-1 differs from ICAM-1 at the C-terminus, and these changes confer solubility to sICAM-1 (see US patent No. 5,821,341).

A nucleotide sequence of human sICAM-1 cDNA to mRNA sequence (1443 bases) is, for example, disclosed in US patent No. 5,821,341.
SEQ ID NO. 1: 
1 atggctccca gcagcccccg gcccgcgctg cccgcactcc tggtcctgct cggggctctg
61 ttcccaggac ctggcaatgc ccagacatct gtgtccccct caaaagtcat cctgccccgg
121 ggaggctccg tgctggtgac atgcagcacc tcctgtgacc agcccaagtt gttgggcata
181 gagaccccgt tgcctaaaaa ggagttgctc ctgcctggga acaaccggaa ggtgtatgaa
241 ctgagcaatg tgcaagaaga tagccaacca atgtgctatt caaactgccc tgatgggcag
301 tcaacagcta aaaccttcct caccgtgtac tggactccag aacgggtgga actggcaccc
361 ctcccctctt ggcagccagt gggcaagaac cttaccctac gctgccaggt ggagggtggg
421 gcaccccggg ccaacctcac cgtggtgctg ctccgtgggg agaaggagct gaaacgggag
481 ccagctgtgg gggagcccgc tgaggtcacg accacggtgc tggtgaggag agatcaccat
541 ggagccaatt tctcgtgccg cactgaactg gacctgcggc cccaagggct ggagctgttt
601 gagaacacct cggcccccta ccagctccag acctttgtcc tgccagcgac tcccccacaa
661 cttgtcagcc cccgggtcct agaggtggac acgcagggga ccgtggtctg ttccctggac
721 gggctgttcc cagtctcgga ggcccaggtc cacctggcac tgggggacca gaggttgaac
781 cccacagtca cctatggcaa cgactccttc tcggccaagg cctcagtcag tgtgaccgca
841 gaggacgagg gcacccagcg gctgacgtgt gcagtaatac tggggaacca gagccaggag
901 acactgcaga cagtgaccat ctacagcttt ccggcgccca acgtgattct gacgaagcca
961 gaggtctcag aagggaccga ggtgacagtg aagtgtgagg cccaccctag agccaaggtg
1021 acgctgaatg gggttccagc ccagccactg ggcccgaggg cccagctcct gctgaaggcc
1081 accccagagg acaacgggcg cagcttctcc tgctctgcaa ccctggaggt ggccggccag
1141 cttatacaca agaaccagac ccgggagctt cgtgtcctgt atggcccccg actggacgag
1201 agggattgtc cgggaaactg gacgtggcca gaaaattccc agcagactcc aatgtgccag
1261 gcttggggga acccattgcc cgagctcaag tgtctaaagg atggcacttt cccactgccc
1321 atcggggaat cagtgactgt cactcgagat cttgagggca cctacctctg tcggccagg
1381 agcactcaag gggaggtcac ccgcaagccc cccggtatga gattgtcatc atcactgtgg
1441 tag
 with the corresponding amino acid sequence (SEQ ID NO. 2):

1 MAPSSPRPAL PALLVLLGAL FPGPGNAQTS VSPSKVILPR GGSVLVTCST SCDQPKLLGI
61 ETPLPKKELL LPGNNRKVYE LSNVQEDSQP MCYSNCPDGQ STAKTFLTVY WTPERVELAP
121 LPSWQPVGKN LTLRCQVEGG APRANLTVV LLRGEKELKRE PAVGEPAEVT TTVLVRRDHH
181 GANFSCRTEL DLRPQGLELF ENTSAPYQLQ TFVLPATPPQ LVSPRVLEVD TQGTVVCSLD
241 GLFPVSEAQV HLALGDQRLN PTVTYGNDSF SAKASVSVTA EDEGTQRLTC AVILGNQSQE
301 TLQTVTIYSF PAPNVILTKP EVSEGTEVTV KCEAHPRAKV TLNGVPAQPL GPRAQLLLKA
361 TPEDNGRSFS CSATLEVAGQ LIHKNQTREL RVLYGPRLDE RDCPGNWTWP ENSQQTPMCQ
421 AWGNPLPELK CLKDGTFPLP IGESVTVTRD LEGTYLCRAR STQGEVTRKP PGMRLSSSLW

ICAM-1 is mainly expressed on the endothelial cell surface. It serves as a counter-receptor for the lymphocyte function-associated antigen (LFA-1) and facilitates leukocyte adhesion and endothelial transmigration (Hubbard *et al.,* 2000).

Its soluble form, sICAM-1, was first described in a clinical setting in the early 90s, when discussed in the context of diabetes, cardiovascular and inflammatory diseases (Ridker *et al.,,* 1998). In the following years, it has gained attraction as a biomarker for pancreatitis, obesity and obstructive sleep apnea, subarachnoidal hemorrhage, hepatocellular carcinoma, and endothelial dysfunction or infectious diseases and sepsis (Page *et al.,* 2013; Palmefors *et al.,* 2014; Zhu *et al.,* 2012; Nadeem *et al.,* 2013; Kubo *et al.,* 2008; Mack *et al.,* 2002; Zhu *et al.,* 2013). None of these studies, some of which investigated extremely painful conditions, stratified for pain levels. Importantly, some studies already reported elevated sICAM-1 levels in cluster headache, inflammatory and neuropathic pain, and painful bladder syndrome, undermining a role in painful diseases (Rehmal *et al.,* 2008; Parkitny *et al.,* 2013; Corcoran *et al.,* 2013).

As discussed above, the present invention provides soluble intercellular adhesion molecule-1 (sICAM-1) for use in the diagnosis, monitoring and/or therapy control of pain.

Said use comprises the determination of the pain level or pain intensity.

In one embodiment, the monitoring and/or therapy control of pain comprises monitoring of pain treatment and pain management.

Preferably, the pain is
- chronic pain, such as chronic pain syndrome, chronic low back pain, complex regional pain syndrome, neuropathic pain, postoperative pain, headaches, facial pain, tumor induced pain, wound pain, abdominal pain, musculo-sceletal pain, fibromyalgia,
   or
- acute pain.

Preferably, sICAM-1 is a pain biomarker independent of inflammation.

Said use comprises preferably the determination of sICAM-1 concentration or level in patient specimen,
preferably serum concentration or level of sICAM-1.

The patient specimen is preferably serum or whole blood.

The patent specimen can also be exhaled air (breath) or other tissue, such as saliva or urine

Preferably, sICAM-1 serum concentration or levels are determined with a sensitivity of 83% and a specificity of 76 %,
preferably at sICAM-1 serum levels of about 105 to about 120 ng/ml serum, preferably about 110 to about 115 ng/ml serum, such as about 112.8 ng/ml serum.

This allows distinguishing or classifying patients into patients with no pain or mild pain versus patients with moderate to severe pain,
preferably using a cut-off value of about 105 to about 120 ng sICAM-1 /ml serum, preferably about 110 to about 115 ng sICAM-1 /ml serum, such as about 112.8 ng sICAM-1 /ml serum.

Preferably, the patients are
- patients in narcosis / under anesthetic or sedated (perioperative, intensive care),
- patients with no or limited communication (dementia, children, delirium, consciousness disorder),
- patients where self-reported pain values may be doubtful (such as in a conflict of interest setting), and/or
- non-human patients (veterinary medicine, animal models).

In a preferred embodiment, the concentration or level of sICAM-1 is determined using analysis of molecular weight, anti-sICAM-1 antibodies or sICAM-1-binding antibody fragments (such as Fab, scFv, di-scFv, tri-scFv, T-cell engagers, microantibodies, among others),
optionally labeled, such as with chromophors, fluorophors, radioirotopes, magnetic labels, or combinations thereof.

Preferably, determining the concentration or level of sICAM-1 comprises the use of
- immunoassays
   such as ELISA, Multiplex/ELISA,
- colorimetric and/or fluorimetric assays,
- radioactive (immune)assays,
- lateral flow immunochromatographic assay (LFIA),
- magnetic immunoassay,
- strip test or assay,
- Lab-on-a-Chip systems (LOCs),
- mass spectrometry,
- chromatography,
or combinations thereof.

The use of sICAM-1 according to the present invention preferably comprises laboratory of point-of-care testing of pain and/or pain intensity,
such as at home, in intensive care, perioperatively, at a nursing home, special care facility, at a veterinary clinic, or in animal model experiments.

The use of sICAM-1 according to the present invention further preferably comprises guidance in medical, survey, or forensic reports to verify pain-related claims.

### Diagnostic methods

As discussed above, the present invention provides a method for the diagnosis, monitoring and/or therapy control of pain.

Said method comprises the step of
determining the concentration or level of sICAM-1 in patient specimen.

Said method preferably allows the detection of pain about 10 min after onset and up to one hour afterwards.

The patient specimen is preferably serum or whole blood.

The patent specimen can also be exhaled air (breath) or other tissue, such as saliva or urine

The method of the present invention comprises in one embodiment the determination of the pain level or pain intensity.

Preferably, the monitoring and/or therapy control of pain comprises monitoring of pain treatment and pain management.

Preferably, the pain is
- chronic pain, such as chronic pain syndrome, chronic low back pain, complex regional pain syndrome, neuropathic pain, postoperative pain, headaches, facial pain, tumor induced pain, wound pain, abdominal pain, musculo-sceletal pain, fibromyalgia,
   or
- acute pain.

Preferably, sICAM-1 serum concentration or levels are determined with a sensitivity of 83% and a specificity of 76 %,
preferably at sICAM-1 serum levels of about 105 to about 120 ng/ml serum, preferably about 110 to about 115 ng/ml serum, such as about 112.8 ng/ml serum.

Thus, patients can be distinguished or classified into patients with no pain or mild pain versus patients with moderate to severe pain,
preferably using a cut-off value of about 105 to about 120 ng sICAM-1 /ml serum, preferably about 110 to about 115 ng sICAM-1 /ml serum, such as about 112.8 ng sICAM-1 /ml serum.

Preferably, the patients are
- patients in narcosis / under anesthetic or sedated (perioperative, intensive care),
- patients with no or limited communication (dementia, children, delirium, consciousness disorder),
- patients where self-reported pain values may be doubtful (such as in a conflict of interest setting), and/or
- non-human patients (veterinary medicine, animal models).

In a preferred embodiment, the concentration or level of sICAM-1 is determined using analysis of molecular weight, anti-sICAM-1 antibodies or sICAM-1-binding antibody fragments (such as Fab, scFv, di-scFv, tri-scFv, T-cell engagers, microantibodies,), optionally labeled, such as with chromophors, fluorophors, radioirotopes, magnetic labels, or combinations thereof.

Preferably, determining the concentration or level of sICAM-1 comprises the use of
- immunoassays
   such as ELISA, Multiplex/ELISA,
- colorimetric and/or fluorimetric assays,
- radioactive (immune)assays,
- lateral flow immunochromatographic assay (LFIA),
- magnetic immunoassay,
- strip test or assay,
- Lab-on-a-Chip systems (LOCs),
- mass spectrometry,
- chromatography,
or combinations thereof.

The method of the present invention preferably comprises laboratory or point-of-care testing of pain and/or pain intensity,
such as at home, in intensive care, perioperatively, at a nursing home, special care home/facility, at a veterinary clinic, or in animal model experiments.

### Further description of preferred embodiments

### - Backgnound

Pain therapy is strongly guided by patients' self-reporting and unspecific clinical variables. When self-reporting is not an option, assessment of pain intensity is challenging. There is currently no biomarker that may help to guide pain management.

### - Methods

In this observational, single-center study, we investigated 30 serum cytokine levels for a potential use as surrogate markers for pain intensity in a screening set of 71 chronic pain patients and 24 healthy controls. Measurements were repeated in 53 patients at a follow-up visit. After identification of soluble Intercellular Adhesion Molecule-1 (sICAM-1) as a potential biomarker for pain in the screening- and follow-up set, we prospectively validated its discriminatory capability in 21 newly recruited subjects.

### - Findings

sICAM-1 was significantly associated with patient-reported pain intensity (correlation r = 0.43, 95% CI: 0.22 - 0.61, P < 0.001). It yielded differential serum levels in patients rating "no or mild", "moderate", and "severe" pain, and served as a good predictor of pain intensity (AUCs: 0.79 - 0.85). Similar results were obtained on follow-up and validation data.

Sensitivity and specificity to discern healthy controls and/or patients with mild pain from patients with moderate/severe pain at a cut-off of 112.8 ng/ml serum-sICAM-1 ranged from 0.77 - 1.0 and 0·57 - 1.0, respectively. Changes in pain ratings after treatment correlated with changes in sICAM-1 levels.

### - Interpretation

In this study, sICAM-1 serum levels were significantly associated with self-reported pain intensity in chronic pain patients. Our findings demonstrate that sICAM-1 can be used as a biomarker for pain intensity.

### - Discussion

Despite a plethora of technological advances in modern medicine, measurement of pain is still limited to a patient's ability to self-report or judgment of clinical parameters such as heart rate and blood pressure. Recent literature suggests a crosstalk between pain and inflammation (Alexander *et al.,* 2012; Sun *et al.,* 2013; Koch *et al.,* 2007). Therefore, we investigated a set of 30 inflammation- and pain-related biomarkers to search for potential capability as a biomarker of pain. We found that soluble Intercellular Adhesion Molecule-1 (sICAM-1) measured in serum correlates with patients self-reported pain levels and could reliably discern between patients with no or mild pain and patients suffering from moderate to severe pain.

In predictive modeling, sICAM-1 outperformed clinical parameters such as heart rate and mean arterial pressure. We estimated sensitivity and specificity at sICAM-1 serum levels of 112.8 ng/ml to be 0.83 and 0.76, respectively. Discriminatory potency was similar among patients with chronic low back pain, complex regional pain syndrome, and patients with other types of pain. Additionally, changes in sICAM-1 levels correlated with changes in pain intensity. We tested a variety of variables for potential confounding or effect modification, using different strategies, such as model adjustment, propensity scores, and stratification.

ICAM-1 is mainly expressed on the endothelial cell surface. It serves as a counter-receptor for the lymphocyte function-associated antigen (LFA-1) and facilitates leukocyte adhesion and endothelial transmigration (Hubbard *et al.,* 2000). Machelska *et al.* (2002), for example, described ICAM-1 as a key molecule involved in the recruitment of opioid-containing leukocytes.

Its soluble form, sICAM-1, was first described in a clinical setting in the early 90s, when discussed in the context of diabetes, cardiovascular and inflammatory diseases (Ridker *et al*.,, 1998). In the following years, it has gained attraction as a biomarker for pancreatitis, obesity and obstructive sleep apnea, subarachnoidal hemorrhage, hepatocellular carcinoma, and endothelial dysfunction or infectious diseases and sepsis (Page *et al.,* 2013; Palmefors *et al.,* 2014; Zhu *et al.,* 2012; Nadeem *et al.,* 2013; Kubo *et al.,* 2008; Mack *et al.,* 2002). For example, Doupis *et al.* (2009) found that diabetic patients with peripheral neuropathy had increased serum levels of inflammatory cytokines and change in the levels of various growth factors. Patients with painful neuropathy had a further increase in inflammation, as indicated by higher levels of CRP, and endothelial dysfunction, as indicated by the levels of sICAM (Doupis *et al.,* 2009).

None of these studies, some of which investigated extremely painful conditions, stratified for pain levels. Importantly, some studies already reported elevated sICAM-1 levels in cluster headache, inflammatory and neuropathic pain, and painful bladder syndrome, undermining a role in painful diseases (Rehmal *et al.,* 2008; Parkitny *et al.,* 2013; Corcoran *et al.,* 2013).

Our study is the first to show that sICAM-1 levels correlate with self-reported pain intensity across different chronic pain syndromes, and may allow clinicians to distinguish between patients with no or mild pain from patients with moderate to severe pain. The current study does not allow us to draw inferences on the nature of the relationship between sICAM-1 and pain. Serum levels may not necessarily reflect the sensation of pain, but may be the result of a secondary process, as is the case with other routinely used clinical surrogate markers. A previous work Rutkowski *et al.* (2002) found sICAM-1 to be involved in pain mediation in rats with induced lumbar radiculopathy. In this context, sICAM-1 serum levels might reflect the body's reaction to a painful stimulus.

This study clearly shows that sICAM-1 can serve as a biomarker predicting a painful condition that needs intervention when patients are unable to reliably self-report their pain.

The following examples and drawings illustrate the present invention without, however, limiting the same thereto.

### BRIEF DESCRIPTION OF THE DRAWINGS

### Figure 1.

**A)** CONSORT flow diagram of the study population.
**B)** The automatically inferred Bayesian network structure that was used to detect potential confounders.

### Figure 2

A) Comparison of sICAM-1 to IL-6 in the screening and follow-up set. Values were normalized to the median expression of the healthy control subjects. For IL-6, only values above the limit of detection were considered for calculating the median expression.
**B)** Areas under the receiver-operating-curve for sICAM-1 in order to discriminate 1. healthy controls from patients, 2. healthy controls and patients with mild pain from patients with moderate and severe pain, and 3. patients with mild from patients with moderate to severe pain. The color-coded, shaded areas correspond to the 95% confidence intervals.
**C)** Relative changes of sICAM-1 correlate with relative changes in pain intensity. The dotted lines represent the 95% confidence interval of the regression line.
**D)** AUCs for different pain types; shaded areas represent the 95% confidence intervals for each receiver-operating-characteristic
**E)** self-reported pain intensity versus sICAM-1 levels show significant correlation
**F)** Clinical markers of pain for no or mild pain (o) and moderate to severe pain (+). Values are normalized to the respective median value of the no or mild pain group.

### EXAMPLES

### Example 1 Materials & Methods

### 1.1 Study Design and Population

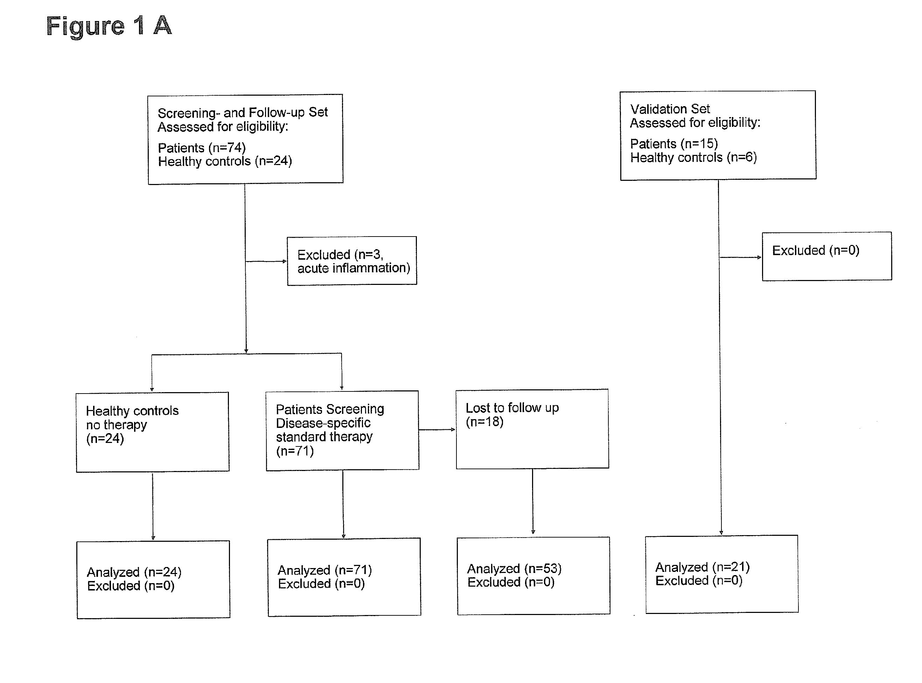

The study followed the principles of the Declaration of Helsinki and was approved by the Ethics Committee of the Ludwig-Maximilians-University Munich. The study was designed as an observational, case-control, single center study to assess predictive capability of 30 cytokines to predict pain intensity levels. Chronic pain patients and healthy controls fulfilling the inclusion criteria were recruited within 18 months, starting 9/2011 (screening set). Patients available to follow-up after six months were re-analyzed to assess internal validity (follow-up set). After identification of sICAM-1 as a potential surrogate marker of pain intensity, we prospectively recruited 15 new patients and 6 healthy control subjects to validate our results (validation set). All patients of at least 18 years of age visiting the Department of Anesthesiology and Pain Medicine, University Hospital of Munich for chronic pain were considered eligible to participate in this study. Exclusion criteria were a recently started medication with opioids, non-opioids or co-analgetics, treatment or prior history of treatment with immunomodulatory agents, any signs of chronic or acute inflammatory disease (such as fever, abnormal total or differential leukocyte count, and increased plasma-concentration of C-reactive protein), autoimmune, neoplastic, or psychiatric disease, drug abuse, pregnancy, and a major impairment of renal or liver function (Consort-Flow Diagram see Figure 1A). In order to avoid bias introduced by the type of pain, patients with neuropathic, nociceptive, and mixed pain as well as patients with complex regional pain syndrome were included.

### 1.2 Pain Syndromes

Non-specific chronic low back pain (CLBP) was defined as persistent low back pain not attributable to a recognizable specific pathology (e.g. infection, tumor, osteoporosis, trauma, inflammatory disorder or radicular syndrome). Diagnosis of a Complex Regional Pain Syndrome I (CRPS) was made when a patient met the "Budapest Criteria" and suffered from unilateral symptoms in a limb (Harden 2010). Patients with other types of chronic pain included polyneuropathy, postherpetic neuralgia, orofascial pain, mixed pain and musculoskeletal pain.

### 1.3 Assessment of Pain intensity and Stress

Patients rated their current maximum pain intensity at rest and at movement using an 11-point numeric rating scale (NRS), ranging from "no pain" (0) to "worst pain imaginable" (10). Mild pain was defined as the range 0 - 3, moderate pain as 4 - 6, and severe pain as 7 - 10 points. This classification was based on the distribution of pain intensity scores of patients declaring that the pain was unbearable to them (minimum NRS=3, median NRS=6, maximum NRS=10). Self-perceived stress was evaluated using the German version of the Questionnaire for Actual Demands ("KAB": "Kurzfragebogen zur aktuellen Beanspruchung"). The KAB was designed to repeatedly quantify an individual's acute or chronic distress. It is highly sensitive to short-term or situational changes during a stressful experience. The rating is based on a 6-point scale ranging from 1 to 6 based on normalized adjectives. Higher KAB values (rating scale, 1 - 6) indicate increased perceived levels of distress (Gauter-Fleckenstein *et al.,* 2007).

### 1.4 Determination of Cytokines

Blood samples were collected, centrifuged and stored in polypropylene aliquot tubes at -80°C until examination. Serum levels of 30 pro- and anti-inflammatory cytokines were measured by Rules-Based Medicine, (Austin, Texas, USA), using the Human Cytokine multiplex immunoassay (Human Cytokine Map A+B). The multiplex microbead assay is based on Luminex technology and measures proteins in a similar manner to standard sandwich ELISA, with comparable sensitivity and range (Chowdhurry *et al.,* 2009; Breen *et al,* 2011). Measurements below the limit of detection were set to 50% of the detection limit. Cytokines were excluded from subsequent analysis, when the great majority of measurements were below the limit of detection.

### 1.5 Statistical Analysis

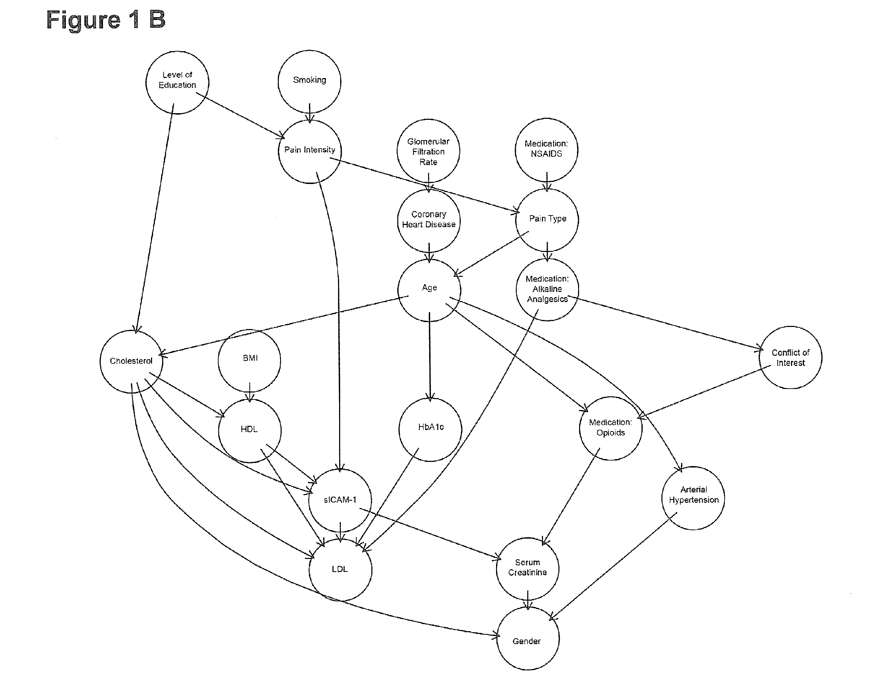

We used the open source statistical software R for statistical analysis. Using a random simulation model, we estimated the required sample size for detection of a biomarker at a power between 50% (standard deviation 10% of the mean) and 90% (standard deviation 5% of the mean) to be n=70 patients and 23·3 healthy controls. Initial analyses had been carried out on the screening set (first recruitment period); results were tested on a follow-up set and an unseen validation set (second recruitment period). Comparisons between two groups were performed using the Mann-Whitney-U-test. We tested for a non-normal distribution via the Kolmogorov-Smirnov test. For multi-group comparisons the Kruskal-Wallis-test was used. The resulting p-values were adjusted to control for a false discovery rate of 5% based on the Benjamini-Hochberg algorithm. If significant, the Mann-Whitney-U was used as a post-hoc test. Correlation was assessed using Pearson's product-moment correlation. Throughout the study, a significance level of P < 0.05 was set. All markers were evaluated in terms of expression differences between control (no pain), mild, moderate, and severe pain. Predictive potential of the remaining candidate cytokines was evaluated in terms of significance in and discriminatory power of a univariate logistic regression model to discern a) patients from healthy controls, b) healthy controls and patients with mild pain versus patients with moderate and severe pain, and c) patients with mild versus those with moderate and severe pain. Calibration was evaluated by calculating Hosmer-Lemeshow's goodness-of-fit statistic for g=5, 10, and 15 groups. In order to assess the relation between changes in pain intensity and biomarker values, the relative change was calculated. Confounding was assessed by testing the impact of gender, age, pain type, level of education, body mass index, history of coronary heart disease, total cholesterol, high- and low density lipoprotein, smoking, renal function (measured by glomerular filtration rate and creatinine serum levels), conflict of interest, and prior pain medication on the estimated sICAM-1 effect in a logistic regression model. Additionally, the structure of a Bayesian network was learned using the bnlearn package (Figure 1B). Effect modification was evaluated by assessing the significance of the interaction term with the median-binarized variable. Conflict of interest was defined as immediate and obvious personal or financial benefit for the patient, if the condition persists. Level of education was coded as "1": no school degree, "2": high school degree without additional training, "3": high school degree with additional training, "4": college degree, "5": university degree. Propensity scores were matched using nearest-neighbor matching. After matching, scores were tested for normality and a paired t-test was performed to avoid significant differences in propensity score distribution (P > 0.9).

### Example 2 Results

### 2.1 Study Design and Population

A total of 457 new patients visited our pain department in the recruitment period of 18 months. Of these, 74 patients were considered eligible for our study. Three of these patients were excluded after eligibility assessment, but prior to blood sampling because of symptoms of an acute infection during physical exam. 24 healthy subjects devoid of acute or chronic pain (assessment via questionnaire) were recruited in parallel. The resulting screening cohort included 35 men of age 21 - 83 years and 60 women of age 20 - 77 years. Of these 71 chronic pain patients, 53 patients were available for follow-up measurements 6 months after the initial assessment (Table 1). After identification of sICAM-1 as a potential surrogate marker of pain intensity, we prospectively recruited 15 new patients and 6 healthy control subjects. The validation cohort consisted of 5 men of age 26 - 66 years and 16 women of age 19 - 78 years.

| **Table 1. Patient Demographics^{§}** | | | |
|---|---|---|---|
| **Variable** | **Screening Set** | **Follow-up Set** | **Validation Set** |
| Total number | 95 | 53 | 21 |
| Patients | | | |
| Number | 71 | 53 | 15 |
| Age | 47 (20 - 83) | 47(21 - 77) | 58 (19 - 78) |
| Gender | 48:23 (f:m) | 34:19 (f:m) | 13:2 (f:m) |
| Level of education | 3 (1 - 5) | 3 (2-5) | 3.5 (1 - 5) |
| Smoking | 20 | 16 | 3 |
| Pain intensity | 5 (0 - 10) | 5 (0 - 10) | 8 (2 - 10) |
| Pain types | | | |
| CLBP | 40 | 37 | 2 |
| CRPS | 9 | 2 | 6 |
| Other | 22 | 14 | 7 |
| Control subjects | | | |
| Number | 24 | 0 | 6 |
| Age | 41 (23 - 55) | NA | 31 (26 - 45) |
| Gender | 12:12 (f:m) | NA | 3:3 (f:m) |
| Level of education | 4.5 (3-5) | NA | 4.5 (2-5) |
| Smoking | 2 | NA | 0 |

| | | | |
|---|---|---|---|
| § CLBP: chronic low back pain, CRPS: complex regional pain syndrome, Other: other types of chronic pain syndromes; where possible, values are reported as median (range); f:m = female:male | | | |

### 2.2 Identification of candidate markers

Eight of 30 cytokines measured were below detection limits (Table 2).

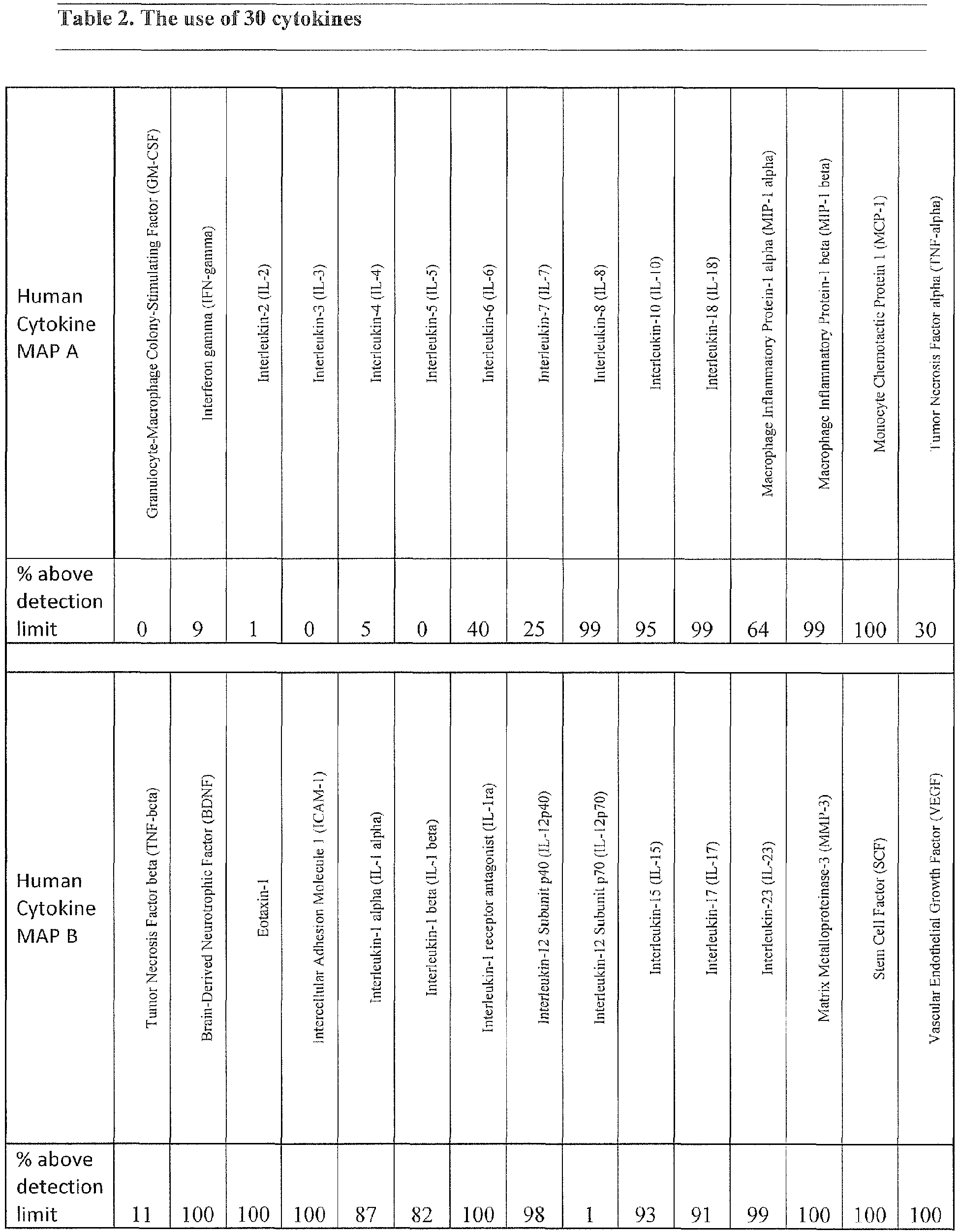

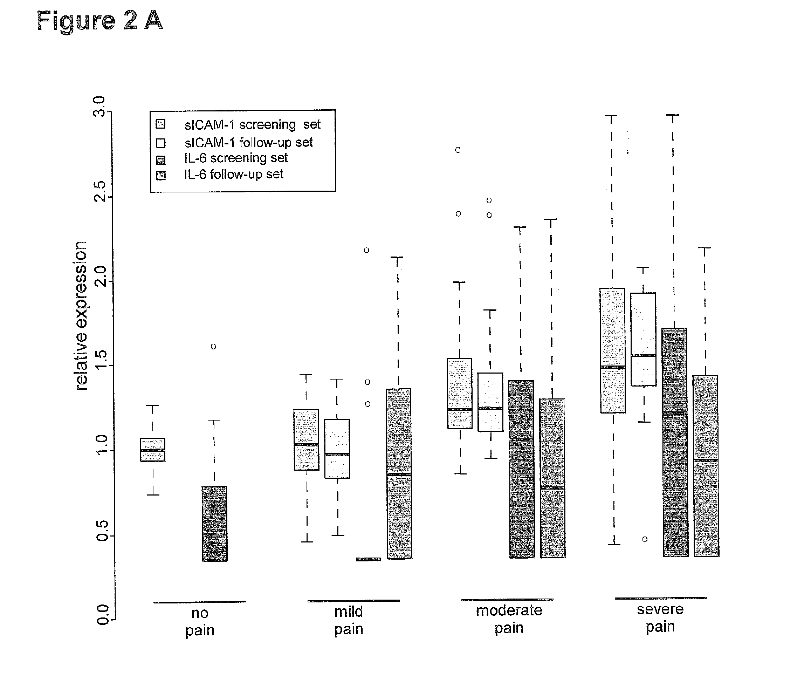

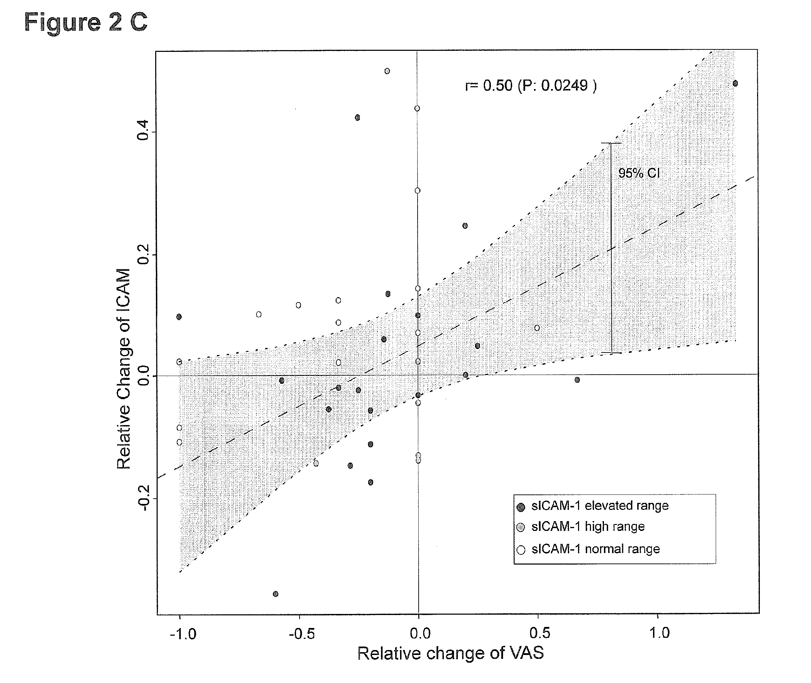

Therefore, we analyzed serum levels of the 22 detectable cytokines in 71 patients suffering from mild to severe pain and 24 healthy control subjects. Of these molecules, IL-6 and sICAM-1 showed differential serum levels between patients with mild and patients with moderate or severe pain (Figure 2A; Table 3). sICAM-1 was the only candidate that yielded significant correlation between the self-reported pain level and measured serum levels in patients (correlation r = 0.43, 95% CI: 0.22 - 0.61, P < 0.001).

| **Table 3. Inter-Group comparison of sICAM-1 and IL-6^{§}** | | |
|---|---|---|
| **Pain Level** | **IL-6^{∀}** | **sICAM-1** |
| control vs mild pain | 0.8 vs 0.8 (P: 0.58) | 101.5 vs 104 (P: 0.72) |
| mild vs moderate pain | 0.8 vs 2.4 (P: 0.01) | 104 vs 124·5 (P: 0.009) |
| moderate pain vs control | 2.4 vs 0.8 (P: 0.004) | 124·5 vs 101·5 (P < 0·001) |
| moderate vs severe pain | 2.4 vs 2.6 (P: 0.65) | 124·5 vs 150 (P: 0.03) |
| severe vs mild pain | 2.6 vs 0.8 (P: 0.02) | 150 vs 104 (P < 0.001) |
| severe pain vs control | 2.6 vs 0.8 (P: 0.006) | 150 vs 101·5 (P < 0.001) |

| | | |
|---|---|---|
| § Median value group 1 versus median value group 2 (p-value) ∀ Values below the limit of detection (1.6ng/ml) were set to 50% (0.8 ng/ml) | | |

### 2.3 Development of a Biomarker Panel

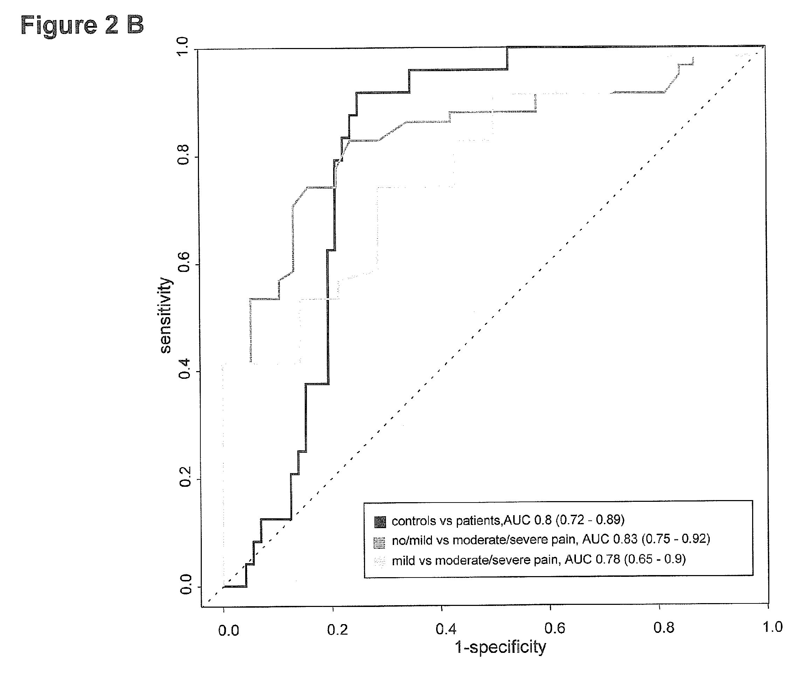

We grouped study subjects into those with no (controls) and mild versus those with moderate and severe pain in order to design a biomarker panel based on a logistic regression model. Using penalized stepwise logistic regression, the model contained sICAM-1, IL-6, IL-23 as covariate predictors in order to discern healthy controls from patients, as well as to distinguish no or mild pain from moderate to severe pain. Only sICAM-1 remained to discriminate between mild versus moderate and severe pain. Since a significant amount of IL-6 measurements were below the limit of detection, we also defined a model of IL-6 levels and a binary variable indicating if IL-6 levels were measurable. This model revealed that measurability is more predictive of pain than IL-6 levels themselves. Univariate models based on sICAM-1 to predict pain intensity appear to be well calibrated (P: 0.11 - 0.72), yielding AUCs of 0.85 and 0.79 (Figure 2B).

### 2.4 Derivation and Evaluation of an sICAM-1 Cut-off

sICAM-1 serum levels were normally distributed in the control group. We therefore defined a cut-off value one standard deviation from the mean, resulting in 112.8 ng/ml. Sensitivity and specificity were calculated for the full screening set, the reduced screening set including patients only, the follow-up set, and the validation set consisting of patients and control subjects. Sensitivity ranged from 0.77 (follow-up set) - 1.0 (validation set) and specificity from 0.57 (follow-up set) - 1.0 (validation set).

We estimated the odds ratio for identifying moderate to severe pain to be 11.65 (95% CI 3.80 - 41.67). This was based on the aforementioned cut-off value in the full screening set from a logistic regression model. The model was adjusted for level of education, cholesterol, and HDL, as a result of the confounder analysis (see "2.7 Assessment of Confounding", below).

### 2.5 Reevaluation and Correlation Analysis between sICAM-1 and Pain Intensity Changes

In order to assess internal validity, sICAM-1 levels were remeasured in 53 patients from the screening set that were available for follow-up analysis after conservative therapy. Both, the univariate models as well as the combined model of sICAM-1 and IL-6 were evaluated on this data, yielding similar results as in the screening cohort (Figure 2A). We also assessed the association between changes in pain and changes in sICAM-1 values. Overall, there was a medium correlation between changes in self-reported pain and changes in sICAM-1 serum levels (r=0.33, P: 0.02). We then investigated patients with and without a potential conflict of interest separately, as it might affect the reported pain intensity. Stratification yielded a similar correlation for patients without conflict of interest (r = 0.37, n = 39, P: 0.02), and no significant correlation for patients with conflict (r = 0.15, n = 14, P: 0.63).

### 2.6 Analysis of Subsets

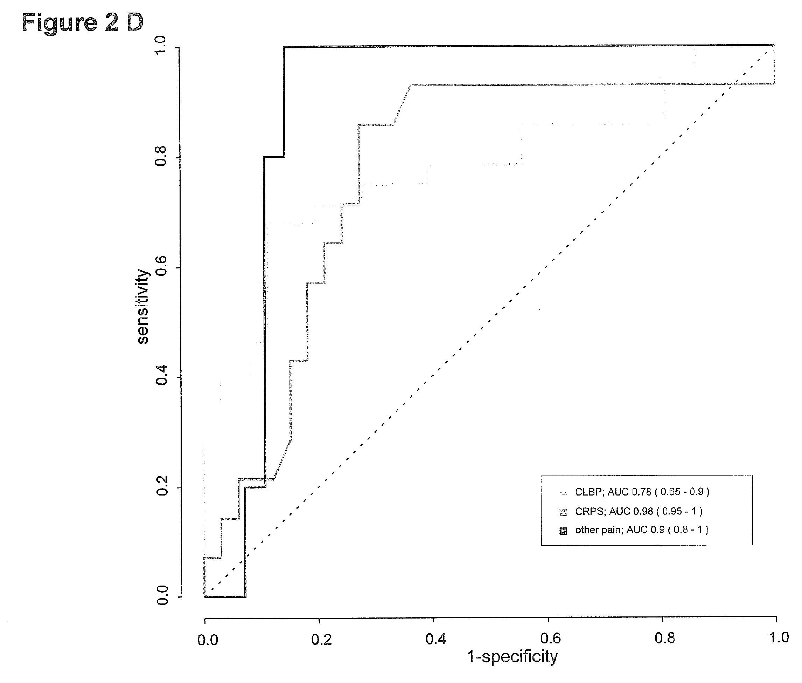

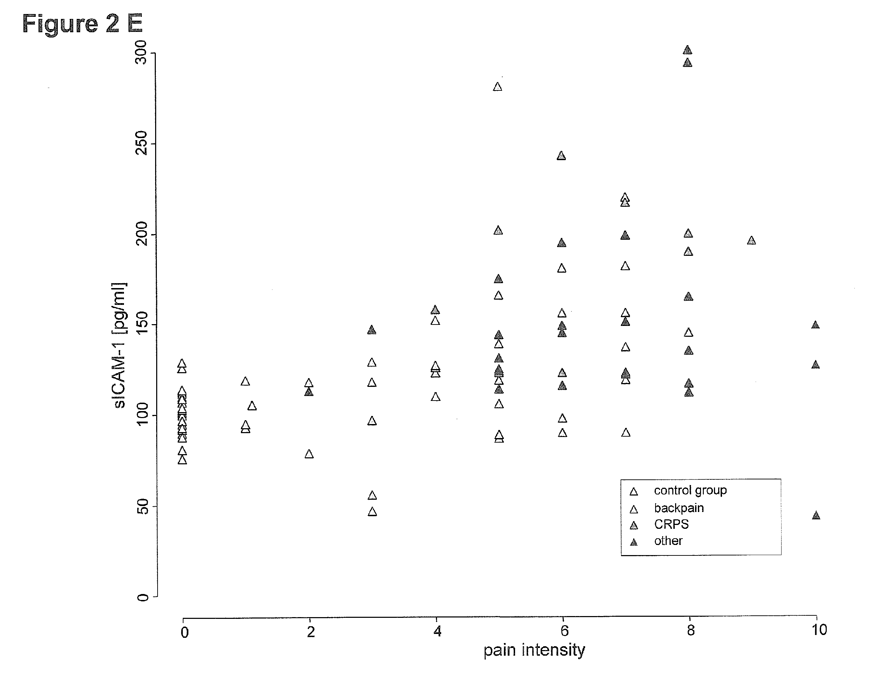

We subgrouped patients in our study into patients with chronic low back pain, complex regional pain syndrome, and other types of pain (mostly neuropathic). These diseases are assumed to have different underlying mechanisms of pathology. We therefore investigated, if there were substantial differences in the discriminative power of sICAM-1 based on pain syndromes, revealing a lower AUC in patients with chronic low back pain than in other types of pain (CLBP: 0.78 (0.65 - 0.90), CRPS: 0.98 (0.95 - 1.00), other pain: 0.95 (0.89 - 1.00)). However, we found great overlap in the 95% confidence intervals of the ROC curves (Figure 2D). Figure 2E illustrates pain levels versus sICAM-1 serum levels, suggesting a positive relationship between pain and sICAM-1 for all subgroups.

### 2.7 Assessment of Confounding

Since sICAM-1 levels have been associated with cardiovascular risk, inflammation, diabetes, and obesity, we first tested a set of potentially confounding variables for significant differences in distribution between healthy controls and patients (Ridker *et al.,* 1998; Page *et al.,* 2013; Gu *et al.,* 2012; Palmefors *et al.,* 2014). Healthy control subjects had less prior pain medication, were younger with a higher level of education, and were less likely smokers. When stratifying for pain intensity, smokers showed an insignificant tendency towards lower sICAM-1 values.

Next, we investigated all variables in a multivariate logistic regression model to evaluate their impact on the estimated effect of sICAM-1 (Table 4). None of the variables affected the estimated sICAM-1-effect (0.06, 95% CI 0.05 - 0.09 in the univariate model) significantly. Smoking, HbA_{1C}, level of education, and conflict of interest significantly contributed to the model. Level of education impacted the estimated sICAM-1 effect the most.

| **Table 4. Assessment of Confounding^{§}** | | | |
|---|---|---|---|
| **Variable** | | **sICAM-1** | **Interaction** |
| Name | p-value | Coefficient | p-value |
| Coronary heart disease | 0.86 | 0.060 | 0.99 |
| Smoking** | 0.007 | 0.059 | 0.82 |
| Arterial hypertension | 0.35 | 0.060 | 0.80 |
| Gender | 0.46 | 0.058 | 0.48 |
| Conflict of interest* | 0.03 | 0.060 | 0.41 |
| Medication with NSAIDs | 0.51 | 0.061 | 0.93 |
| Medication with opioids | 0.99 | 0.059 | > 0.99 |
| Medication with alkaline drugs | 0.99 | 0.056 | > 0.99 |
| Age | 0.07 | 0.056 | 0.64 |
| Body mass index | 0.54 | 0.056 | 0.63 |
| Level of education* | 0.03 | 0.049 | 0.76 |
| HbA_{1C}* | 0.04 | 0.063 | 0.75 |
| Creatinine | 0.91 | 0.060 | 0.60 |
| Total cholesterol | 0.72 | 0.059 | 0.88 |
| Low Density Lipoprotein | 0.74 | 0.057 | 0.39 |
| High Density Lipoprotein | 0.09 | 0.062 | 0.67 |

| | | | |
|---|---|---|---|
| § Parameters estimated from a multivariate model on the full screening set; sICAM-1 coefficient 95% confidence interval: 0·04 - 0·09; NSAIDs: Non-steroidal anti-inflammatory drugs, HbA_{1C}: glycosylated hemoglobin A_{1C} */** parameter significantly contributed to the multivariate model (* P < 0·05; ** P < 0·01) | | | |

We furthermore learned the structure of a bayesian belief network to investigate more complex structural relationships between variables (See Figure 1B). In accordance with the multivariate modeling, the network structure suggests that level of education may serve as a potential confounder, since it influences pain intensity as well as sICAM-1 levels (via cholesterol and HDL). We then built a propensity score matching model on these variables on patients with mild, and patients with moderate to severe pain. The estimated AUC on a propensity-score matched cohort (n=12 mild pain and n=12 moderate to severe pain) was 0.76 (95% CI 0.56 - 0.95). Effect modification was tested by the introduction and evaluation of interaction terms. None of the tested variables displayed significant effect modification.

### 2.8 Comparison to clinical markers

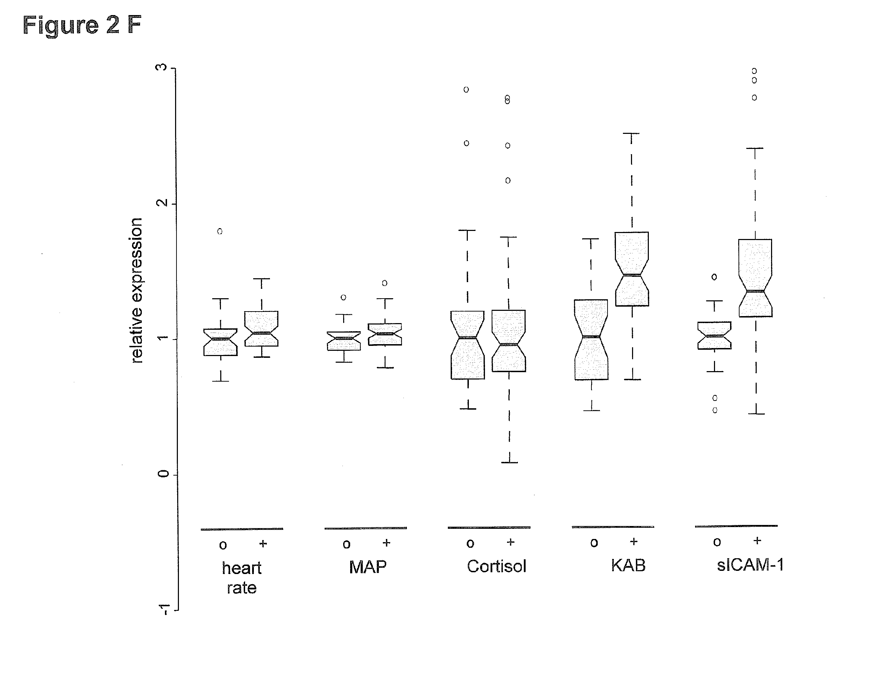

We evaluated the clinical parameters heart rate and mean arterial blood pressure which may help to estimate the pain intensity of patients who are not able to report it themselves. Even though heart rate and mean arterial pressure showed an increased median value in patients with moderate to severe pain, the difference was not significant. Assessment of distress measured by serum cortisol levels and a psychological questionnaire for assessment of current distress revealed no significantly different cortisol levels, but significantly higher KAB scores in moderate to severe pain (Figure 2F). In linear and logistic regression modeling, KAB scores did not significantly contribute to sICAM-1 serum levels in predicting pain intensity in patients, while it did significantly enhance discrimination between patients and controls.

| **Table 5. Inter-Group comparison of three potential biomarkers⁸** | | | | | | |
|---|---|---|---|---|---|---|
| **Model** | **sIC AM-1** | | **IL-6** | | **IL**-**23** | |
| | AUC (95% CI) | coefficient (95% CI) | AUC (95% CI) | coefficient (95 %CI) | AUC (95% CI) | coefficient (95% CI) |
| *univariate* | | | | | | |
| control + patients | 0.85 (0.77 - 0.93) | 0.06** (0.04 - 0.09) | 0.72 (0.62 - 0.81) | 0.66** (0.29 - 1.10) | 0.61 (0.50-0.73) | 1.14* (0.09 - 2.28) |
| patients only | 0.79 (0.66 - 0.92) | 0.04** (0.02 - 0.07) | 0.72 (0.57 - 0.86) | 0.57* (0.10-1.22) | 0.49 (0.34 - 0.64) | -0.048 (-1.46 - 1.39) |
| *multivariate* | | | | | | |
| control + patients | 0.89 (0.82 - 0.95) | 0.050** (0.02 - 0.08) | 0.89 (0.82-0.95) | 0.75** (0.24 - 1.37) | 0.89 (0.82 - 0.95) | 1.88* (0.36 - 3.57) |

| | | | | | | |
|---|---|---|---|---|---|---|
| §AUC = area under the receiver operator characteristic; * P < 0·05, ** P < 0·01 | | | | | | |

The features disclosed in the foregoing description, in the claims and/or in the accompanying drawings may, both separately and in any combination thereof, be material for realizing the invention in diverse forms thereof.

### REFERENCES

Alexander GM, Peterlin BL, Perreault MJ, Grothusen JR, Schwartzman RJ. Changes in plasma cytokines and their soluble receptors in complex regional pain syndrome. J Pain. 2012; 13(1): 10-20.
Barkin RL, Barkin SJ, Barkin DS. Perception, assessment, treatment, and management of pain in the elderly. Clin Geriatr Med. 2005; 21(3): 465-90.
Breen EC, Reynolds SM, Cox C, et al. Multisite comparison of high-sensitivity multiplex cytokine assays. Clin Vaccine Immunol. 2011; 18(8): 1229-42.
Chanques G, Jaber S, Barbotte E, et al. Impact of systematic evaluation of pain and agitation in an intensive care unit. Crit Care Med. 2006; 34(6): 1691-9.
Chowdhury F, Williams A, Johnson P. Validation and comparison of two multiplex technologies, Luminex and Mesoscale Discovery, for human cytokine profiling. J Inmnunol Methods. 2009; 340(1): 55-64.
Corcoran AT, Yoshimura N, Tyagi V, Jacobs B, Leng W, Tyagi P. Mapping the cytokine profile of painful bladder syndrome/interstitial cystitis in human bladder and urine specimens. World J Urol. 2013; 31(1): 241-6.
Escallier KE, Nadelson MR, Zhou D, Avidan MS. Monitoring the brain: processed electroencephalogram and peri-operative outcomes.Anaesthesia. 2014; 69(8):899-910.
Ellis A, Bennett DL. Neuroinflammation and the generation of neuropathic pain. Br J Anaesth. 2013; 111(1): 26-37.
Gauter-Fleckenstein B, Kaviani R, Weiss C, et al. [Perioperative patient management. Evaluation of subjective stress and demands of patients undergoing elective gynaecological surgery]. Anaesthesist. 2007; 56(6): 562-70.
Gu HF, Ma J, Gu KT, Brismar K. Association of intercellular adhesion molecule 1 (ICAM1) with diabetes and diabetic nephropathy. Front Endocrinol (Lausanne). 2012; 3: 179.
Harden RN. Objectification of the diagnostic criteria for CRPS. Pain Med. 2010; 11(8): 1212-5.
Hubbard AK, Rothlein R. Intercellular adhesion molecule-1 (ICAM-1) expression and cell signaling cascades. Free Radic Biol Med. 2000; 28(9): 1379-86.
Joffe AM, Hallman M, Gelinas C, Herr DL, Puntillo K. Evaluation and treatment of pain in critically ill adults. Semin Respir Crit Care Med. 2013; 34(2): 189-200.
Koch A, Zacharowski K, Boehm O, et al. Nitric oxide and pro-inflammatory cytokines correlate with pain intensity in chronic pain patients. Inflamm Res. 2007; 56(1): 32-7.
Kubo Y, Ogasawara K, Kakino S, et al. Serum inflammatory adhesion molecules and high-sensitivity C-reactive protein correlates with delayed ischemic neurologic deficits after subarachnoid hemorrhage. Surg Neurol. 2008; 69(6): 592-6; discussion 6.
Lees JG, Duffy SS, Moalem-Taylor G. Immunotherapy targeting cytokines in neuropathic pain. Front Pharmacol. 2013; 4: 142.
Lien CA, Kopman AF. Current recommendations for monitoring depth of neuromuscular blockade. Curr Opin Anaesthesiol. 2014; 27(6):616-22.
Machelska H, Mousa SA, Brack A, et al. Opioid control of inflammatory pain regulated by intercellular adhesion molecule-1. J Neurosci. 2002; 22(13): 5588-96.
Mack WJ, Mocco J, Hoh DJ, et al. Outcome prediction with serum intercellular adhesion molecule-1 levels after aneurysmal subarachnoid hemorrhage. J Neurosurg. 2002; 96(1): 71-5.
Nadeem R, Molnar J, Madbouly EM, et al. Serum inflammatory markers in obstructive sleep apnea: a meta-analysis. J Clin Sleep Med. 2013; 9(10): 1003-12.
Page AV, Liles WC. Biomarkers of endothelial activation/dysfunction in infectious diseases. Virulence. 2013; 4(6): 507-16.
Palmefors H, DuttaRoy S, Rundqvist B, Borjesson M. The effect of physical activity or exercise on key biomarkers in atherosclerosis--a systematic review. Atherosclerosis. 2014; 235(1): 150-61.
Payen JF, Bosson JL, Chanques G, Mantz J, Labarere J. Pain assessment is associated with decreased duration of mechanical ventilation in the intensive care unit: a post Hoc analysis of the DOLOREA study. Anesthesiology. 2009; 111(6): 1308-16.
Parkitny L, McAuley JH, Di Pietro F, et al. Inflammation in complex regional pain syndrome: a systematic review and meta-analysis. Neurology. 2013; 80(1): 106-17.
Remahl AI, Bratt J, Mollby H, Nordborg E, Waldenlind E. Comparison of soluble ICAM-1, VCAM-1 and E-selectin levels in patients with episodic cluster headache and giant cell arteritis. Cephalalgia. 2008; 28(2): 157-63.
Ridker PM, Hennekens CH, Roitman-Johnson B, Stampfer MJ, Allen J. Plasma concentration of soluble intercellular adhesion molecule 1 and risks of future myocardial infarction in apparently healthy men. Lancet. 1998; 351(9096): 88-92.
Rutkowski MD, Winkelstein BA, Hickey WF, Pahl JL, DeLeo JA. Lumbar nerve root injury induces central nervous system neuroimmune activation and neuroinflammation in the rat: relationship to painful radiculopathy. Spine (Phila Pa 1976). 2002; 27(15): 1604-13.
Sun JM, Sun LZ, Liu J, Su BH, Shi L. Serum interleukin-15 levels are associated with severity of pain in patients with knee osteoarthritis. Dis Markets. 2013; 35(3): 203-6.
Wang H, Schiltenwolf M, Buchner M. The role of TNF-alpha in patients with chronic low back pain-a prospective comparative longitudinal study. Clin J Pain. 2008; 24(3): 273-8.
Zhu HH, Jiang LL. Serum inter-cellular adhesion molecule 1 is an early marker of diagnosis and prediction of severe acute pancreatitis. World J Gastroenterol. 2012; 18(20): 2554-60.
Zhu XW, Gong JP. Expression and role of icam-1 in the occurrence and development of hepatocellular carcinoma. Asian Pac J Cancer Prev. 2013; 14(3): 1579-83.
Zwakhalen SM, Hamers JP, Abu-Saad HH, Berger MP. Pain in elderly people with severe dementia: a systematic review of behavioural pain assessment tools. BMC Geriatr. 2006; 6: 3.

## Claims

1. Soluble intercellular adhesion molecule-1 (sICAM-1) for use in the diagnosis, monitoring and/or therapy control of pain.

2. The sICAM-1 for use according to claim 1, comprising the determination of the pain level or pain intensity,
and/or wherein the monitoring and/or therapy control of pain comprises monitoring of pain treatment and pain management.

3. The sICAM-1 for use according to claim 1 or 2, wherein the pain is
- chronic pain, such as chronic pain syndrome, chronic low back pain, complex regional pain syndrome, neuropathic pain, postoperative pain, headaches, facial pain, tumor induced pain, wound pain, abdominal pain, musculo-sceletal pain, fibromyalgia,
or
- acute pain.

4. The sICAM-1 for use according to any one of claims 1 to 3, comprising the determination of sICAM-1 concentration or level in patient specimen,
preferably serum concentration or level of sICAM-1,
wherein the patient specimen is preferably serum or whole blood, exhaled air (breath) or other tissue, such as saliva or urine.

5. The sICAM-1 for use according to any one of claims 1 to 4, wherein sICAM-1 serum concentration or levels are determined with a sensitivity of 83% and a specificity of 76 %, preferably at sICAM-1 serum levels of about 105 to about 120 ng/ml serum, preferably about 110 to about 115 ng/ml serum, such as about 112.8 ng/ml serum.

6. The sICAM-1 for use according to claim 5, wherein patients can be distinguished or classified into patients with no pain or mild pain versus patients with moderate to severe pain, preferably using a cut-off value of about 105 to about 120 ng sICAM-1 /ml serum, preferably about 110 to about 115 ng sICAM-1 /ml serum, such as about 112.8 ng sICAM-1 /ml serum.

7. The sICAM-1 for use according to any one of claims 1 to 6, wherein the patients are
- patients in narcosis / under anesthetic or sedated,
- patients with no or limited communication,
- patients where self-reported pain values may be doubtful, and/or
- non-human patients.

8. The sICAM-1 for use according to any one of claims 1 to 7, wherein the concentration or level of sICAM-1 is determined using analysis of molecular weight, anti-sICAM-1 antibodies or sICAM-1-binding antibody fragments (such as Fab, scFv, di-scFv, tri-scFv, T-cell engagers, microantibodies,),
optionally labeled, such as with chromophors, fluorophors, radioirotopes, magnetic labels, or combinations thereof,
wherein determining the concentration or level of sICAM-1 preferably comprises the use of
- immunoassays
such as ELISA, Multiplex/ELISA,
- colorimetric and/or fluorimetric assays,
- radioactive (immune)assays,
- lateral flow immunochromatographic assay (LFIA),
- magnetic immunoassay,
- strip test or assay,
- Lab-on-a-Chip systems (LOCs),
- mass spectrometry,
- chromatography,
or combinations thereof.

9. The sICAM-1 for use according to any one of claims 1 to 8, comprising laboratory or point-of-care testing of pain and/or pain intensity,
such as at home, in intensive care, perioperatively, at a nursing home, special care facility, at a veterinary clinic, or in animal model experiments.

10. A method for the diagnosis, monitoring and/or therapy control of pain, comprising the step of
determining the concentration or level of sICAM-1 in patient specimen,
preferably serum concentration or level of sICAM-1,
wherein the patient specimen is preferably serum or whole blood, exhaled air (breath) or other tissue, such as saliva or urine.

11. The method of claim 10, allowing the detection of pain about 10 min after onset and up to one hour afterwards,
and/or comprising laboratory or point-of-care testing of pain and/or pain intensity,
such as at home, in intensive care, perioperatively, at a nursing home, special care facility, at a veterinary clinic, or in animal model experiments.

12. The method of claim 10 or 11, wherein sICAM-1 serum concentration or levels are determined with a sensitivity of 83% and a specificity of 76 %,
preferably at sICAM-1 serum levels of about 105 to about 120 ng/ml serum, preferably about 110 to about 115 ng/ml serum, such as about 112.8 ng/ml serum,
wherein preferably patients can be distinguished or classified into patients with no pain or mild pain versus patients with moderate to severe pain,
more preferably using a cut-off value of about 105 to about 120 ng sICAM-1 /ml serum, preferably about 110 to about 115 ng sICAM-1 /ml serum, such as about 112.8 ng sICAM-1 /ml serum.

13. The method of any one of claims 10 to 12, wherein the concentration or level of sICAM-1 is determined using analysis of molecular weight, anti-sICAM-1 antibodies or sICAM-1-binding antibody fragments (such as Fab, scFv, di-scFv, tri-scFv, T-cell engagers, microantibodies,),
optionally labeled, such as with chromophors, fluorophors, radioirotopes, magnetic labels, or combinations thereof,
wherein determining the concentration or level of sICAM-1 preferably comprises the use of
- immunoassays
such as ELISA, Multiplex/ELISA,
- colorimetric and/or fluorimetric assays,
- radioactive (immune)assays,
- lateral flow immunochromatographic assay (LFIA),
- magnetic immunoassay,
- strip test or assay,
- Lab-on-a-Chip systems (LOCs),
- mass spectrometry,
- chromatography,
or combinations thereof.

14. The method of any one of claims 10 to 13, wherein the patients are
- patients in narcosis / under anesthetic or sedated,
- patients with no or limited communication,
- patients where self-reported pain values may be doubtful, and/or
- non-human patients,
and/or wherein the pain is
- chronic pain, such as chronic pain syndrome, chronic low back pain, complex regional pain syndrome, neuropathic pain, postoperative pain, headaches, facial pain, tumor induced pain, wound pain, abdominal pain, musculo-sceletal pain, fibromyalgia,
or
- acute pain.

15. The method of any one of claims 10 to 14, comprising the determination of the pain level or pain intensity,
and/or wherein the monitoring and/or therapy control of pain comprises monitoring of pain treatment and pain management.
